# EUROPEAN PATENT APPLICATION

(11) **EP 4 763 983 A1**
(43) Date of publication of application: **24.06.2026**
(21) Application number: 24855726.6
(22) Date of filing: 16.08.2024
(51) Int. Cl.: C12N 15/10, C12N 15/63, C12P 19/34

(54) **METHOD FOR PURIFYING NUCLEIC ACID MOLECULES**

(30) Priority: 18.08.2023 CN 202311049806
(71) Applicant: Zhenjiang Probio Biotech Co., Ltd., Zhenjiang, Jiangsu 212000 (CN)
(72) Inventor: CHEN, Junpeng, Zhenjiang, Jiangsu 212000 (CN); WANG, Xu, Zhenjiang, Jiangsu 212000 (CN)
(74) Representative: Synergy IP Group AG
(86) International application number: PCT/CN2024/112594
(87) International publication number: WO 2025/040001

(57) **Abstract**

Provided is a method for purifying nucleic acid molecules, specifically relating to a method for purifying circular nucleic acid molecules. The purification method involves few chromatography steps and has a high total yield%, and using the purification method to prepare LcDNA or linear DNA allows for a short production cycle and low production costs.

## Description

### Cross Reference to Related Applications

The present application claims the right of priority for Chinese patent application no. CN 202311049806.5 filed on August 18, 2023, which is incorporated herein by reference in its entirety.

### Technical Field

The present invention relates to a method for purifying nucleic acid molecules, specifically relating to a method for purifying circular nucleic acid molecules.

### Background Art

Linear plasmid DNA, due to the advantages of its molecular structure, enables more efficient *in vitro* transcription (IVT) and has been widely used as a template for *in vitro* transcription of mRNA. Linear closed DNA (LcDNA), as it does not carry prokaryotic replicons and resistance gene sequences but only carries the gene sequence of interest, is widely used as a non-viral gene delivery vector.

mRNA is an emerging class of drugs for the prevention and treatment of various diseases. In recent years, the successful commercialization of two mRNA vaccines against the novel coronavirus COVID-19, produced by Modema and BioNtech respectively, highlights the great potential of mRNA technology to revolutionize life sciences and pharmaceutical research. The development of mRNA technology has stimulated the application of *in vitro* transcribed mRNA (IVT mRNA) in numerous preclinical studies and clinical trials. Currently, mRNA drugs are mainly applied in three therapeutic areas: immunotherapy, protein replacement therapy, and regenerative medicine therapy. Specifically, these can be divided into cancer immunotherapy, vaccines for infectious diseases, vaccines for allergy alleviation, protein replacement therapy, cell fate reprogramming, and genome editing, etc.

The industrial production process for IVT mRNA can be broadly divided into three parts: circular plasmid production, linear plasmid production, and IVT mRNA production. The *in vitro* transcription and purification of mRNA are completely *in vitro,* cell-free processes. Therefore, once a highly standardized production process is developed for the mRNA stage, batch-to-batch reproducibility is easily maintained. Currently, the difficulties and challenges in the industrial production of mRNA mainly focus on the large-scale industrial production of the starting materials, i.e., circular plasmid DNA and linear plasmid DNA. The current relatively mature production process from circular plasmid DNA to linear plasmid DNA sequentially includes: bacterial fermentation, bacterial cell harvest, bacterial cell lysis, clarification and concentration of lysate, a classic three-step chromatography process (gel filtration chromatography, affinity chromatography, and anion exchange chromatography), concentration and liquid exchange of circular plasmid, digestion, anion exchange chromatography, concentration and buffer exchange, sterilization and filtration, and linear plasmid filling. However, the complex and lengthy process steps of the entire production process significantly increase the cost, cycle, and process instability of large-scale mRNA industrial production.

Therefore, there remains a need for more optimized processes for the industrial production of mRNA.

### Summary

In a first aspect, the present invention provides a method for purifying a nucleic acid molecule, the method comprises:
1) providing a suspension comprising the nucleic acid molecule; and
2) loading the suspension from step 1 onto a porous polyacrylamide membrane and performing capture, to obtain the nucleic acid molecule.

In some embodiments, the suspension in step 1) comprises a NaCl solution at a final concentration of 50 - 150 mM.

In some embodiments, the method further comprises, prior to the loading of step 2), a step of adding an equilibration buffer to the porous polyacrylamide membrane.

In some embodiments, the method further comprises, after the loading of step 2), a step of adding an equilibration buffer to the porous polyacrylamide membrane.

In some embodiments, the equilibration buffer is a mixed solution of 1M KAc and 150 mM NaCl.

In some embodiments, the method further comprises, after the capture of step 2), a step of performing gradient elution on the obtained nucleic acid molecule and collecting an eluate comprising the nucleic acid molecule.

In some embodiments, a buffer for the gradient elution is a mixed solution of 100 mM Tris and 1M NaCl in an elution gradient of 20%.

In some embodiments, the collecting an eluate is collecting an eluate when the UV value at UV280 or UV260 is greater than or equal to 200 mAU.

In some embodiments, the porous polyacrylamide membrane is a high-density strong anion porous polyacrylamide membrane, and in some embodiments, the high-density strong anion porous polyacrylamide membrane is a Natrix Q membrane.

In some embodiments, the nucleic acid molecule is a circular nucleic acid molecule, and preferably circular DNA.

In some embodiments, the circular nucleic acid molecule is a plasmid comprising at least one restriction endonuclease digestion site.

In some embodiments, the circular nucleic acid molecule is a plasmid, the plasmid comprises two prokaryotic telomerase digestion sites which are connected in tandem in the same direction, both ends of the prokaryotic telomerase digestion site are independently provided with one or more restriction endonuclease digestion sites, and a gene of interest is inserted between the prokaryotic telomerase digestion sites.

In some embodiments, the restriction endonuclease digestion site at each end of the plasmid is selected from any one or more of digestion sites of: AccI, AflII, AgeI, Apal, AscI, AvrII, BamHI, BglI, BglII, BsaI, BspQI, BstBI, BsteII, ClaI, EcoNI, EcoRI, EcoRV, FseI, HindIII, KpnI, MfeI, MluI, NcoI, NdeI, NheI, NotI, PacI, PstI, PvuI, PvuII, SacI, SacII, SalI, ScaI, SmaI, SpeI, StuI, SwaI, XbaI, XhoI and XmaI.

In some embodiments, the restriction endonuclease digestion site is selected from any one or more of digestion sites of: HindIII, SalI, SmaI, BamHI, EcoRV, XbaI, KpnI and EcoRI.

In a second aspect, the present invention provides a method for preparing LcDNA, the method comprises:
a) providing a plasmid obtained by the method according to the first aspect, and preforming digestion with a prokaryotic telomerase;
b) preforming digestion with at least two restriction endonucleases, and preforming further enzymatic digestion with an exonuclease; and
c) polishing the LcDNA containing a gene of interest.

In a third aspect, the present invention provides a method for preparing linear DNA, the method comprises:
a) providing a plasmid obtained by the method according to the first aspect, and preforming digestion with a restriction endonuclease; and
b) polishing the linear DNA containing a gene of interest.

In some embodiments, the method provided in the second or third aspect, prior to the step of digestion, further comprises adjusting the pH of the eluate to pH 7.0-8.0 using a 3M Tris buffer.

In some embodiments, the polishing comprises using a composite resin filler Capto Core 700 for chromatography.

In some embodiments, the chromatography comprises:
a) adding an equilibration buffer to the resin; and
b) loading an enzyme digestion reaction liquid for chromatography and collecting a flow-through liquid;
and the equilibration buffer is a mixed solution of 100 - 300 mM NaCl, 10 mM EDTA and 20 mM Tris-HCl.

In some embodiments, the method further comprises, after the polishing, at least one process selected from concentration, sterilization and filtration, and filling.

In a fourth aspect, the present invention provides use of the LcDNA prepared according to the second aspect or the linear DNA prepared according to the third aspect in the preparation of mRNA.

The beneficial effects of the present invention:
1) the method for purifying a nucleic acid molecule described in the present invention uses a porous polyacrylamide membrane for chromatographic capture, reducing the number of chromatography steps and increasing the total yield%, without affecting the quality of the linear plasmid DNA/LcDNA;
2) the method for purifying a nucleic acid molecule described in the present invention allows the circular plasmid obtained through chromatographic capture to be directly used for digestion without purification, for use in the production of LcDNA or linear DNA. Therefore, the method for preparing LcDNA or linear DNA described in the present invention allows for a short production cycle and low production costs; and
3) the method for preparing LcDNA or linear DNA described in the present invention requires only a single polishing step using a composite filler for chromatography to obtain high-quality linear plasmid DNA or LcDNA, eliminating the need for cumbersome purification steps and resulting in a short production cycle.

### Brief Description of the Drawings

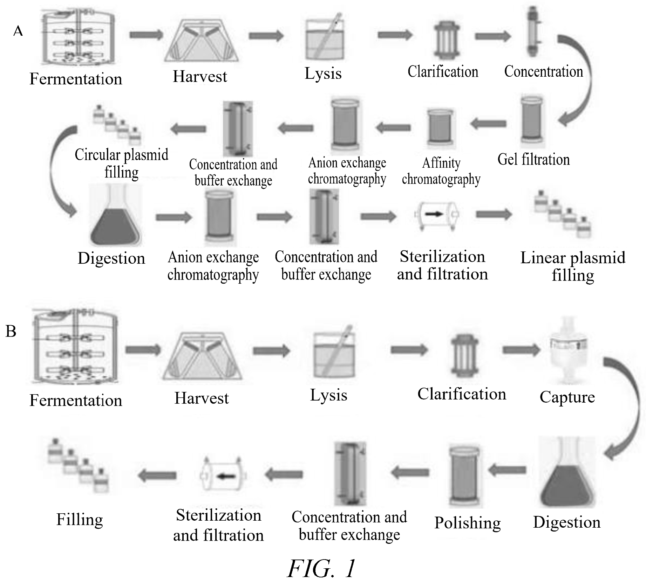

FIG. 1 is a process flow diagram, wherein FIG. 1A is a process flow diagram for conventional linear plasmid DNA or LcDNA; and FIG. 1B is a process flow diagram for linear plasmid DNA or LcDNA according to the present invention.
FIG. 2 is the Natrix Q chromatogram for the first batch of linear plasmid DNA.
FIG. 3 is the Capto Core 700 chromatogram for the first batch of linear plasmid DNA.

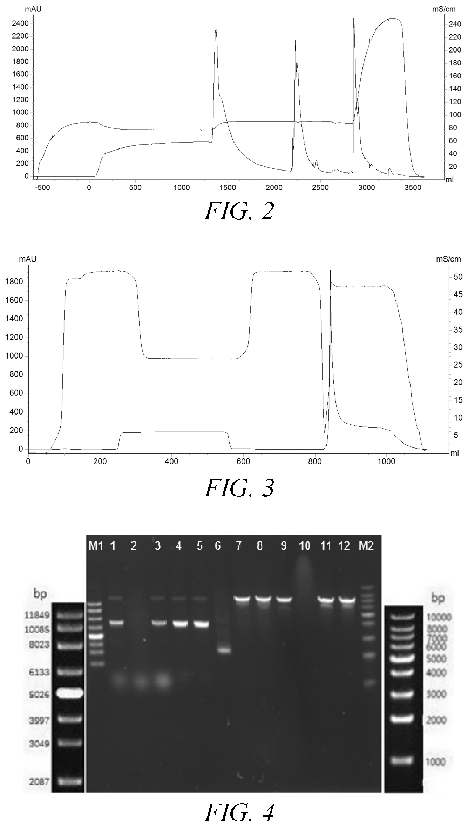

FIG. 4 is the graph of the detection of intermediate samples and the finished product of the first batch of linear plasmid DNA by AGE. Lane M1: Supercoiled DNA control; Lane M2: 1kb DNA control; Lane 1: Natrix Q loading feed liquid; Lane 2: Loading flow-through sample; Lane 3: Equilibrated sample; Lanes 4 and 5: Natrix Q eluted samples; Lane 6: Natrix Q clean sample; Lane 7: XbaI digested sample; Lane 8: Capto Core 700 loading feed liquid; Lane 9: Capto Core 700 eluted sample; Lane 10: Capto Core 700 clean sample; Lane 11: Linear plasmid buffer exchange sample; Lane 12: Finished product of linear plasmid.
FIG. 5 is the Natrix Q chromatogram for the second batch of linear plasmid DNA.
FIG. 6 is the Capto Core 700 chromatogram for the second batch of linear plasmid DNA.

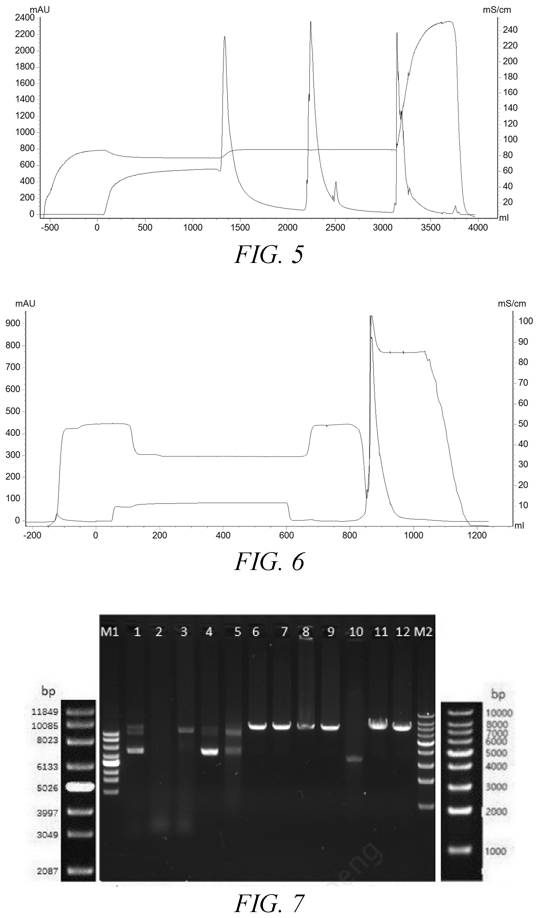

FIG. 7 is the graph of the detection of intermediate samples and the finished product of the second batch of linear plasmid DNA by AGE. Lane M1: Supercoiled DNA control; Lane M2: 1kb DNA control; Lane 1: Natrix Q loading feed liquid; Lane 2: Loading flow-through sample; Lane 3: Equilibrated sample; Lane 4: Natrix Q eluted samples; Lane 5: Natrix Q clean sample; Lane 6: Xbal digested sample; Lane 7: Capto Core 700 loading feed liquid; Lanes 8 and 9: Capto Core 700 eluted sample; Lane 10: Capto Core 700 clean sample; Lane 11: Linear plasmid buffer exchange sample; Lane 12: Finished product of linear plasmid.
FIG. 8 is the Natrix Q chromatogram for the first batch of LcDNA.
FIG. 9 is the Capto Core 700 chromatogram for the first batch of LcDNA.

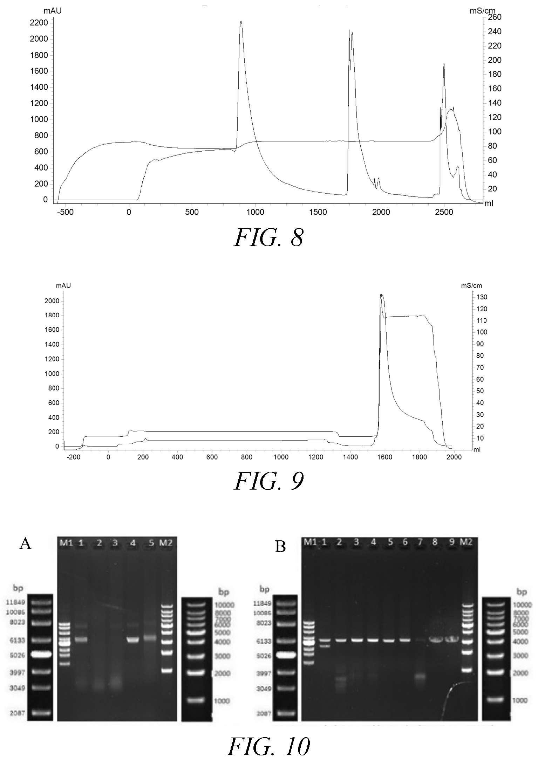

FIG. 10 is the graph of the detection of intermediate samples and the finished product of the first batch of LcDNA by AGE. In FIG. 10A, Lane M1: Supercoiled DNA control; Lane 1: Natrix Q loading feed liquid; Lane 2: Loading flow-through sample; Lane 3: Equilibrated sample; Lane 4: Natrix Q eluted samples; Lane 5: Natrix Q clean sample; In FIG. 10B, Lane M1: Supercoiled DNA control; Lane M2: 1kb DNA control; Lane 1: TelN digested sample; Lane 2: Endonuclease digested sample; Lane 3: Exo III digested sample; Lane 4: Capto Core 700 loading feed liquid; Lanes 5 and 6: Capto Core 700 eluted sample; Lane 7: Capto Core 700 clean sample; Lane 8: LcDNA buffer exchange sample; Lane 9: Finished product of LcDNA; Lane M2: 1kb DNA control.
FIG. 11 is the Natrix Q chromatogram for the second batch of LcDNA.
FIG. 12 is the Capto Core 700 chromatogram for the second batch of LcDNA.

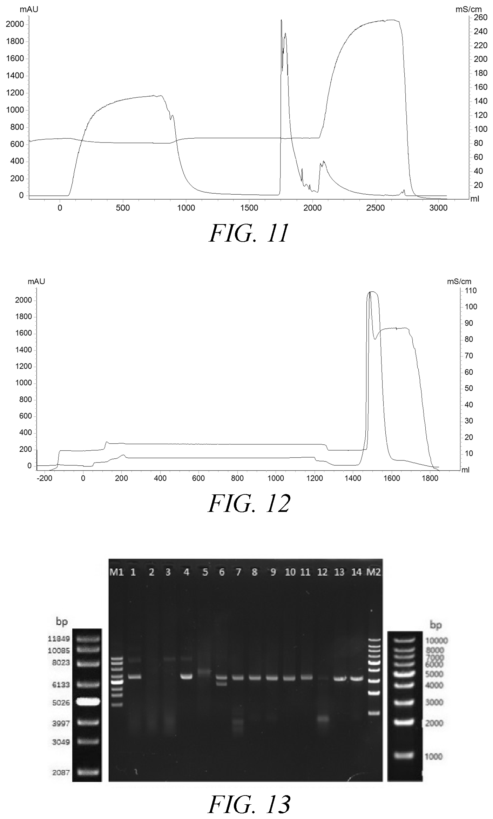

FIG. 13 is the graph of the detection of intermediate samples and the finished product of the second batch of LcDNA by AGE. Lane M1: Supercoiled DNA control; Lane M2: 1kb DNA control; Lane 1: Natrix Q loading feed liquid; Lane 2: Loading flow-through sample; Lane 3: Equilibrated sample; Lane 4: Natrix Q eluted samples; Lane 5: Natrix Q clean sample; Lane 6: TelN digested sample; Lane 7: Endonuclease digested sample; Lane 8: Exo III digested sample; Lane 9: Capto Core 700 loading feed liquid; Lanes 10 and 11: Capto Core 700 eluted sample; Lane 12: Capto Core 700 clean sample; Lane 13: LcDNA buffer exchange sample; Lane 14: Finished product of LcDNA.
FIG. 14 is the Natrix Q chromatogram for the third batch of LcDNA.
FIG. 15 is the Capto Core 700 chromatogram for the third batch of LcDNA.

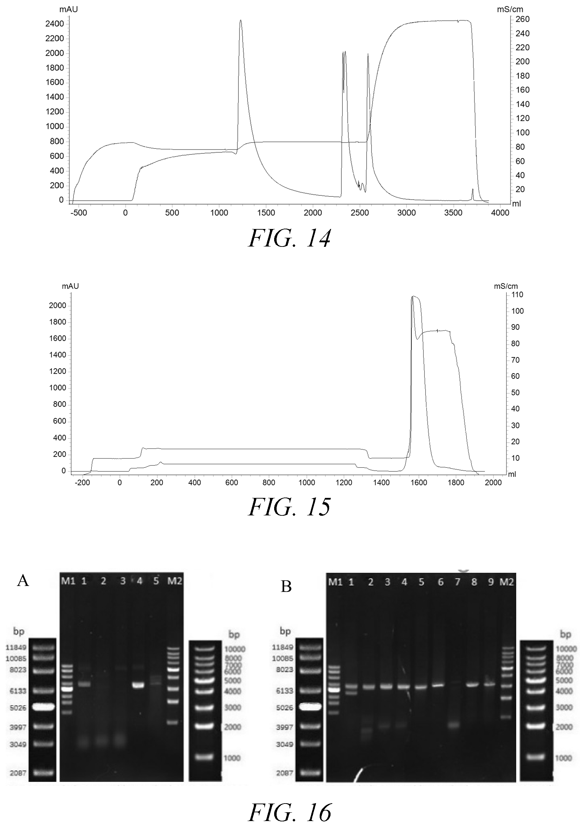

FIG. 16 is the graph of the detection of intermediate samples and the finished product of the third batch of LcDNA by AGE. In FIG. 16A, Lane M1: Supercoiled DNA control; Lane 1: Natrix Q loading feed liquid; Lane 2: Loading flow-through sample; Lane 3: Equilibrated sample; Lane 4: Natrix Q eluted samples; Lane 5: Natrix Q clean sample; Lane M2: 1kb DNA control; In FIG. 16B, Lane M1: Supercoiled DNA control; Lane 1: TelN digested sample; Lane 2: Endonuclease digested sample; Lane 3: Exo III digested sample; Lane 4: Capto Core 700 loading feed liquid; Lanes 5 and 6: Capto Core 700 eluted sample; Lane 7: Capto Core 700 clean sample; Lane 8: LcDNA buffer exchange sample; Lane 9: Finished product of LcDNA; Lane M2: 1kb DNA control.

### Detailed Description of Embodiments

Unless defined otherwise, the technical and scientific terms used herein have the same meaning as commonly understood by a person skilled in the art to which the present invention belongs.

The terms "and/or" and "or/and" used herein are selected to encompass any one of two or more associated items listed therein, as well as any and all combinations of the associated items listed therein, wherein the any and all combinations include combinations of any two of the associated items listed therein, any more of the associated items listed therein, or all of the associated items listed therein. It should be noted that when at least three items are connected by a combination of at least two conjunctions selected from "and/or" and "or/and", it should be understood that in the present application, the technical solutions definitely include the technical solution in which items are all connected by "logic AND" and also definitely include the technical solutions in which items are all connected by "logic OR". For example, "A and/or B" includes the three parallel solutions of A, B and A+B. As another example, the technical solution of "A, and/or, B, and/or, C, and/or, D" includes any one of A, B, C, and D (i.e., the technical solutions in which items are all connected by "logic OR"), also includes any and all combinations of A, B, C, and D, that is, combinations of any two or any three of A, B, C, and D, and further includes the combination of all the four items A, B, C, and D (i.e., the technical solution in which items are all connected by "logic AND").

The terms "contain", "comprise", and "include" used herein are synonymous, inclusive or open-ended, and do not exclude additional, unrecited members, elements, or method steps.

Numerical ranges expressed by endpoints in the present disclosure include all numbers and fractions included within the range, as well as the recited endpoints.

The meaning of the concentration values involved in the present disclosure include fluctuations within a certain range. For example, it can fluctuate within the corresponding precision range. For example, with regard to the value 2%, fluctuations within the range of ±0.1% may be permitted. For larger values or values that do not require too fine control, the meaning is also allowed to include larger fluctuations. For example, with regard to the value 100 mM, fluctuations within the range of ±1%, ±2%, ±5%, etc. may be permitted. When a molecular weight is involved, the meaning of its value is allowed to include fluctuations with the range of ±10%.

In the present disclosure, expressions involving terms such as "a plurality of" and "multiple" refer to a quantity greater than or equal to 2, unless otherwise specified.

In the present disclosure, the technical features described in an open-ended manner include both a closed technical solution consisting of the listed features and an open-ended technical solution comprising the listed features.

In the present disclosure, the terms "preferably", "preferentially", "more preferably", and "appropriately" are solely used for describing better embodiments or examples, and it should be understood that the scope of the present disclosure is not intended to be limited. In the present disclosure, the terms "optionally", "optional" and "alternative" mean that the subject modified by the terms are dispensable, that is, the terms mean that the parallel technical solutions "with" or "without" the subject modified by the terms can both be selected. If the term "alternative" appears multiple times in a technical solution, unless otherwise specified and without any contradictions or mutually restrictive relationships, the term "alternative" is independent in each occurrence.

All documents mentioned in the present disclosure are incorporated in the present application by reference as if each document is cited separately as a reference. The cited documents involved in the present disclosure are incorporated by reference in their entireties for all purposes unless they conflict with the objectives and/or technical solutions of the present application. When a document is cited in the present disclosure, definitions of relevant technical features, terms, nouns, phrases, etc. in the cited document are also incorporated herein. When a document is cited in the present disclosure, examples and preferred modes of the related technical features cited are also incorporated by reference in the present application but are limited to those that enable the implementation of the present disclosure. It should be understood that where a reference conflicts with the description of the present application, the present application shall prevail, or the reference should be modified as appropriate based on the description of the present application.

As used herein, the term "buffer substance" or "buffer" refers to an aqueous solution or composition that resists pH changes when an acid or a base is added to the solution or composition. This resistance to pH changes is due to the buffering properties of such solutions. Therefore, a solution or composition showing buffering activity is referred to as a buffer or a buffer solution. The buffer that can be used in the method of the present disclosure is preferably selected from a phosphate buffer, a phosphate buffered saline buffer (PBS), 2-amino-2-hydroxymethyl-1,3-propanediol (TRIS) buffer, a TRIS buffered saline solution (TBS), TRIS/EDTA (TE), etc.

As used herein, the term "nucleic acid molecule" refers to a molecule comprising more than 3 nucleotides. In some embodiments, the nucleic acid molecule refers to a deoxyribonucleic acid molecule; and in some embodiments, the nucleic acid molecule refers to a ribonucleic acid molecule. In some embodiments, the nucleic acid molecule is linear; and in some embodiments, the nucleic acid molecule is circular. In some embodiments, the nucleic acid molecule is single-stranded and linear; and in some embodiments, the nucleic acid molecule is single-stranded and circular. In some embodiments, the nucleic acid molecule is double-stranded and linear; and in some embodiments, the nucleic acid molecule is double-stranded and circular.

As used herein, the term "circular nucleic acid molecule" refers to a circular deoxyribonucleic acid molecule and a circular ribonucleic acid molecule. In some embodiments, the nucleic acid molecule is single-stranded and circular; and in some embodiments, the nucleic acid molecule is double-stranded and circular. The circular nucleic acid molecule lacks nicks, and includes, but is not limited to: circular DNA, circular RNA, plasmid DNA, covalently closed circular DNA, etc.

In some embodiments, the circular nucleic acid molecule is plasmid DNA. In some embodiments, the plasmid comprises at least one restriction endonuclease site, and preferably two or more different restriction endonuclease sites. The restriction endonuclease site can be used to enzymatically digest the plasmid DNA into linear DNA. In other embodiments, the plasmid comprises two prokaryotic telomerase digestion sites which are connected in tandem in the same direction, and both ends of the prokaryotic telomerase digestion site are independently provided with one or more restriction endonuclease digestion sites. The prokaryotic telomerase digestion site and restriction endonuclease digestion site coexist on the plasmid and are used for digestion to produce LcDNA. A "prokaryotic telomerase digestion site" sequence comprises any complete palindromic sequence, that is, any double-stranded DNA sequence with two-fold rotational symmetry. Methods for producing linear DNA or LcDNA using digestion sites to carry out digestion have been disclosed in multiple documents, such as CN 102301010 B, CN 103080337 A, which are not redundantly described herein.

In some embodiments, the circular nucleic acid molecule is produced by *E. coli.*

A "suspension comprising a nucleic acid molecule" refers to a liquid containing the nucleic acid molecule and other impurities. In some embodiments, the suspension is produced by *E. coli* fermentation. In some embodiments, circular DNA is transformed into *E. coli,* and the circular DNA is amplified through fermentation; after fermentation, the *E. coli* is lysed, and the lysate is clarified; and the obtained supernatant is the suspension comprising the nucleic acid molecule.

The term "capture" refers to a step in a separation procedure. Typically, the capture is the initial step of separation, providing preliminary purification through chromatography, filtration, or other means to obtain the nucleic acid molecule of interest.

In the present invention, the step of capture comprises performing chromatography on the suspension comprising the nucleic acid molecule using a porous polyacrylamide membrane.

The term "purification" as used herein refers to a process of isolating the nucleic acid molecule from the suspension comprising the nucleic acid molecule. In some embodiments, the purification comprises chromatographic capture of the nucleic acid molecule, elution of the nucleic acid molecule, and further polishing. In some embodiments, the chromatography process comprises equilibrating the chromatography column with an equilibration buffer, and loading the sample; and, in some embodiments, equilibrating the sample with the equilibration buffer, and performing gradient elution on the sample. In some embodiments, the equilibration buffer is a mixed solution of 1M KAc and 150 mM NaCl; in some embodiments, the pH of the equilibration buffer is between pH 5-6, for example, pH 5, pH 5.1, pH 5.2, pH 5.3, pH 5.4, pH 5.5, pH 5.6, pH 5.7, pH 5.8, pH 5.9, or pH 6.0; and in some embodiments, the conductivity of the equilibration buffer is between 80-90 mS/cm, for example, 81 mS/cm, 82 mS/cm, 83 mS/cm, 84 mS/cm, 85 mS/cm, 96 mS/cm, 97 mS/cm, 88 mS/cm, 89 mS/cm, or 90 mS/cm. In some embodiments, the volume of the equilibration buffer is 200 times the membrane volume. In some embodiments, the flow rate of the equilibration buffer is 10 membrane volumes per minute.

In some embodiments, the maximum loading capacity for loading the sample is 10 mg/mL. In some embodiments, the loaded suspension comprises a salt solution of 100 - 150 mM NaCl; and in some embodiments, the conductivity of the loaded suspension is between 70-80 mS/cm, such as 70 mS/cm, 71 mS/cm, 72 mS/cm, 73 mS/cm, 74 mS/cm, 75 mS/cm, 76 mS/cm, 77 mS/cm, 78 mS/cm, 79 mS/cm, or 80 mS/cm. In some embodiments, the flow rate for loading the suspension is 10 membrane volumes per minute.

In some embodiments, after the loading is completed, the membrane is equilibrated again using the equilibration buffer.

In some embodiments, the elution buffer is a mixed solution of 100 mM Tris and 1M NaCl. In some embodiments, the pH of the elution buffer is 8.0-9.0; and in some preferred embodiments, the pH of the elution buffer is 9.0. In some embodiments, the volume of the elution buffer is 100 times the membrane volume. In some embodiments, the flow rate of the elution buffer is 5 membrane volumes per minute. In some embodiments, the elution step is carried out by gradient elution, such as in a gradient of 10%, 15%, 20%, 25%, 30%, 35%, 40%, 45%, or 50%.

In some embodiments, the purification process further comprises regenerating the membrane using a regeneration buffer, and the regeneration buffer is a mixed solution of 1M NaOH and 2 M NaCl. In some embodiments, the volume of the regeneration buffer is 200 times the membrane volume. In some embodiments, the flow rate of the regeneration buffer is 10 membrane volumes per minute.

The term "polishing" refers to a process of further purifying the nucleic acid molecule obtained by the chromatographic capture. In some embodiments, the step of polishing is ion exchange chromatography, hydrophobic chromatography, or mixed-mode chromatography. A person skilled in the art can select suitable chromatography methods and fillers as needed. In some embodiments, the chromatography uses a composite resin filler Capto Core 700 for chromatography. In some embodiments, the chromatography comprises:
a) adding an equilibration buffer to the resin; and
b) loading an enzyme digestion reaction liquid for chromatography and collecting a flow-through liquid;
and the equilibration buffer is a mixed solution of 100 - 300 mM NaCl, 10 mM EDTA and 20 mM Tris-HCl. In some embodiments, the collecting the flow-through liquid is collecting a flow-through fraction when the UV value at UV280 or UV260 is greater than or equal to 30 mAU.

The term "LcDNA", also known as linear closed DNA or closed linear DNA, carries the gene of interest and prokaryotic telomerase recognition sequences at its both ends. It has a small size, high safety, and LcDNA exhibits comparable expression levels *in vitro* or *in vivo.*

A "porous polyacrylamide membrane" refers to a membrane primarily composed of polyacrylamide, having a three-dimensional porous hydrogel structure. The porous polyacrylamide membrane preferably has high-density strong anion quaternary amine ligands capable of adsorbing the DNA of interest. In some embodiments, the porous polyacrylamide membrane is a high-density strong anion porous polyacrylamide membrane. In some embodiments, the porous polyacrylamide membrane is a Natrix^{®} Q membrane. In some embodiments, the porous polyacrylamide membrane is a Sartobind^{®} Q membrane.

As used herein, a "gene of interest" refers to any gene that is of interest to experimenters, which is operably linked to a nucleic acid molecule. In some embodiments, the gene of interest is a gene expressing mRNA, a gene expressing siRNA, a gene expressing shRNA, a gene expressing miRNA, or a gene expressing a specific protein. Suitable genes of interest include, but are not limited to, nucleic acids encoding proteins for the treatment of: endocrine, metabolic, hematologic, cardiovascular, neurological, musculoskeletal, urological, pulmonary and immune disorders, including, for example, cancers, inflammatory disorders, immune disorders, and chronic and infectious disorders. The cancers may be, for example, tumours arising from lesions in any of bone, bone junctions, muscle, lung, trachea, heart, spleen, arteries, veins, blood, capillaries, lymph nodes, lymphatic vessels, lymph fluid, oral cavity, pharynx, esophagus, stomach, duodenum, small intestine, colon, rectum, anus, appendix, liver, gallbladder, pancreas, parotid gland, sublingual gland, urinary system, kidney, ureter, bladder, urethra, ovary, fallopian tube, uterus, vagina, vulva, scrotum, testis, vas deferens, penis, eye, ear, nose, tongue, skin, brain, brain stem, medulla oblongata, spinal cord, cerebrospinal fluid, nerve, thyroid, parathyroid, adrenal gland, hypophysis, pineal gland, pancreatic islets, thymus, gonads, sublingual glands and parotid glands; the immune disorders may be, for example, systemic lupus erythematosus, multiple sclerosis, type I diabetes, psoriasis, ulcerative colitis, Sjogren syndrome, scleroderma, polymyositis, rheumatoid arthritis, mixed connective tissue disease, primary biliary cirrhosis, autoimmune haemolytic anaemia, Hashimoto thyroiditis, Addison's disease, vitiligo, Graves' disease, autoimmune myasthenia gravis, ankylosing spondylitis, allergic osteoarthritis, hypersensitivity angiitis, autoimmune neutropenia, idiopathic thrombocytopenic purpura, lupus nephritis, chronic atrophic gastritis, autoimmune infertility, endometriosis, Pasture's disease, pemphigus, discoid lupus erythematosus or dense deposit disease; and the infectious disorders may be any one or any combination of a viral infection, a bacterial infection, a fungal infection and a parasitic infection.

Suitable genes of interest include, but are not limited to, those that encode at least one of the following various proteins: interferons (e.g., IFN-γ, IFN-α, IFN-β, IFN-ω, and IFN-τ); insulin (e.g., Novolin, Humulin, Humalog, Humalog, and Lantus); erythropoietin ("EPO", e.g., epoetin-α, darbepoetin-α, and epoetin-β); antibodies (e.g., monoclonal antibodies, such as rituximab, infliximab, trastuzumab, HumiraTM (adalimumab), omalizumab, tositumomab, RaptivaTM (efalizumab), ErbituxTM (cetuximab), and bevacizumab); antigen-binding fragments, including monoclonal antibodies (e.g., ranibizumab); blood factors (e.g., alteplase, tissue plasminogen activator protein, recombinant human factor VIIa, factor VIIa, factor VIII, factor IX, β-globulin, and haemoglobin); colony stimulating factors (e.g., filgrastim (G-CSF), Neulasta (PEGylated filgrastim), granulocyte colony stimulating factor (G-CSF), granulocyte monocyte colony stimulating factor, macrophage colony stimulating factor, and megakaryocyte colony stimulating factor); growth hormones (e.g., somatotropin and growth hormone); interleukins (e.g., IL-1, IL-2, IL-3, IL-4, IL-5, IL-6, IL-7, IL-8, and IL-9); growth factors (e.g., beclapermin (PDGF), trafermin (bFGF), ancestim (stem cell factor), keratinocyte growth factor, acidic fibroblast growth factor, stem cell factor, basic fibroblast growth factor, and hepatocyte growth factor); soluble receptors (e.g., soluble TNF-α-binding receptors, such as etanercept, soluble VEGF receptors, soluble interleukin receptors, and soluble γ/δT cell receptors); enzymes (e.g., α-glucosidase, β-glucocerebrosidase, and alglucerase), and enzyme activators (e.g., tissue plasminogen activator); chemokines (e.g., IP-10, Mig, Groα/IL-8, RANTES, MIP-1α, MIP-1β, MCP-1, and PF-4); angiogenic agents (e.g., vascular endothelial growth factor (VEGF)); antiangiogenic agents (e.g., soluble VEGF receptors); protein vaccines; neuroactive peptides, such as bradykinin, cholecystokinin, gastrin, secretin, oxytocin, gonadotropin-releasing hormone, β-endorphin, enkephalin, substance P, growth hormone release inhibitory factor, prolactin, galanin, growth hormone releasing hormone, bombesin, dynorphin, neurotensin, motilin, thyrotropin, neuropeptide Y, luteinizing hormone, calcitonin, insulin, glucagon, vasopressin, angiotensin II, thyrotropin releasing hormone, vasoactive intestinal peptide, and sleep-inducing peptide; other proteins, e.g., thrombolytic agents, atrial natriuretic peptide, bone morphogenetic protein, thrombopoietin, relaxin, glial fibrillary acidic protein, follicle-stimulating hormone, human α-1 antitrypsin, leukaemia inhibitory factor, transforming growth factor, insulin-like growth factor, luteinizing hormone, macrophage activating factor, tumour necrosis factor, neutrophil chemotactic factor, nerve growth factor, and tissue inhibitors of metalloproteinases; vasoactive intestinal peptide, angiopoietin, hemangiopoietin, and fibrin; hirudin; leukaemia inhibitory factor; IL-1 receptor antagonists (e.g., anakinra); ion channels, e.g., cystic fibrosis transmembrane conductance regulator protein (CFTR); dystrophin; utrophin (a tumour inhibitor); lysosomal acid α-glucosidase (GAA); *etc.* Suitable genes of interest also include those encoding functional fragments of any of the above proteins; and nucleic acids encoding functional variants of any of the above proteins.

The technical solutions of the present invention will be further described in detail below through examples and in combination with the accompanying drawings. Unless otherwise specified, the methods and materials in the examples described below are conventional products that can be purchased from the market. Those skilled in the art to which the present invention belongs will understand that the methods and materials described below are exemplary only and should not be construed as limiting the scope of the present invention.

### Example 1 Method for preparing and purifying linear DNA

### (1) Bacterial fermentation

Plasmid DNA containing the gene of interest (molecular weight: 3169 bp) (synthesized by GenScript ProBio, vector name: CFL universal, vector molecular weight: 2017 bp) was transformed into *E. coli* for culture and fermentation. The culture temperature of the fermenter was set to 30-37°C, the rotation speed was 200 rpm, and trace elements (components: 6.42 g/L ferric chloride, 4.00 g/L manganese chloride, 1.00 g/L zinc sulphate, 0.40 g/L copper sulphate, 0.50 g/L sodium molybdate, 0.50 g/L boric acid), 0.25 g/L thiamine hydrochloride, and *E. coli* shake flask culture solution (0.1% inoculation ratio) were added proportionally to initiate the fermentation procedure. During the fermentation, the dissolved oxygen content was maintained in the range of 20%-40%. Ammonia water was used as the alkaline liquor to maintain the pH not lower than 6.54. Fermentation was stopped after approximately 24 hours.

### (2) Bacterial cell harvest

After the fermentation was stopped, the bacterial cells were collected by centrifugation using a centrifuge. The rotation speed was set to 6000 - 8000 rpm, the temperature was 4°C, and the centrifugation lasted for 15 - 20 minutes. Depending on the fermentation scale, hollow fibres could also be used to harvest the bacterial cells.

### (3) Bacterial cell lysis

The weight of each of the buffers S1, S2-SDS, S2-NaOH, and S3 in the process of alkaline lysis on bacterial cells was calculated based on the bacterial cell weight, by using the calculation formulas as shown in the table below:

| Bacterial cell weight (A) | | | |
|---|---|---|---|
| Buffer name | Buffer composition | Buffer weight (Kg) | Buffer density (Kg/L) |
| S1 | 50 mM glucose, 25 mM Tris, 10 mM EDTA, pH 8.0 | = A × 10 × 1.004 | 1.004 |
| S2-SDS | 2% SDS | =A × 10 × 1.008 | 1.008 |
| S2-NaOH | 400 mM NaOH | = A × 10 × 1.011 | 1.011 |
| S3 | 3 M potassium acetate | = A × 15 × 1.142 | 1.142 |

The bacterial cells were fully resuspended using the S1 buffer. Then, S2-NaOH and S2-SDS were mixed uniformly in a 1 : 1 volume ratio and poured into the resuspension. The mixture was stirred slowly and gently for 3 - 5 minutes. Finally, the S3 buffer in the proportion indicated in the table above was added. After stirring evenly, the mixture was allowed to stand for 50 - 60 minutes. Depending on the actual conditions of the production facility equipment and production scale, manual lysis or an automated lysis system could be selected for the bacterial cell lysis operation.

### (4) Clarification and filtration of lysate

The lysate after standing was filtered sequentially through a 50 µm filter (Manufacturer: Hangzhou Cobetter Filtration Equipment Co., Ltd., Cat. No.: UFEFL0100050P) and a 0.6 µm filter (Manufacturer: Hangzhou Cobetter Filtration Equipment Co., Ltd., Cat. No.: L30SSCHT06A1P). The filtered supernatant was collected.

### (5) Circular plasmid capture

A NaCl solution at a final concentration of 50 - 150 mM was added to the supernatant obtained in step (4). Subsequently, chromatography was performed using a Natrix Q membrane (Manufacturer: Merck KgaA, Cat. No.: NXF-10, NXF-20, NXF-50; a membrane with an appropriate specification was selected for chromatography according to the actual production scale). The circular plasmids were captured directly from the supernatant. During chromatographic elution, the value at UV260 or UV280 was monitored, and fractions with values greater than or equal to 200 mAU were collected. The parameters of the membrane chromatography process are shown in the table below:

| Natrix Q membrane chromatography | | | | |
|---|---|---|---|---|
| Process step | Line | Volume (membrane volume) | Flow rate (membrane volume/minute) | Composition |
| Equilibration | A1 | 200 times | 10 times | 1M KAc + 150 mM NaCl, pH 5.1, Conductivity 87-89 mS/cm |
| Loading | A2 | Maximum loading capacity 10 mg/mL | 10 times | Filtered lysate supplemented with NaCl at a final concentration of 100 - 150 mM, Conductivity 76-79 mS/cm |
| Post-equilibration | A1 | 200 times | 10 times | 1M KAc + 150 mM NaCl, pH 5.1, Conductivity 87-89 mS/cm |
| Elution | 20% B1 | 100 times | 5 times | 100 mM Tris + 1M NaCl pH 9.0 |
| Regeneration | B2 | 200 times | 10 times | 1M NaOH + 2 M NaCl |

Sartobind^{®} Q membrane could also be used for the chromatography process, but compared to the Natrix Q membrane, its purification effects were poorer, and the membrane was prone to clogging with slower flow rates.

### (6) Circular plasmid digestion

The collected Natrix Q eluate was first adjusted to pH 7.0 - 8.0 using a 3M Tris buffer, and then digestion was performed. The parameters of the digestion process are shown in the table below (using Xba I, digestion system 1000 mL as an example). After sequentially adding and mixing thoroughly the components in the table below, the mixture was incubated in a constant temperature water bath at 37°C for 12 - 16 hours, followed by treatment of digestion at 75°C for 15 - 30 minutes.

| Component | Addition amount |
|---|---|
| Digestion buffer | 100 mL |
| Natrix Q eluate | 40 mg (e.g., concentration 0.2 mg/mL, i.e., 200 mL) |
| Xba I | 1 mL (unit enzyme activity 20000 U/mL) |
| Water for injection | Add up to 1000 mL |

### (7) Chromatographic purification of linear plasmid DNA

The solution obtained in step (6) was first filtered through a 0.2 µm filter membrane, and the filtrate was collected and then loaded onto a chromatography column containing the composite resin filler Capto Core 700 (Manufacturer: Cytiva, Cat. No.: 17-5481-03). The value at UV260 or UV280 was monitored, and fractions with values greater than or equal to 30 mAU were collected. The parameters of the chromatography process are shown in the table below:

| Capto Core 700 chromatography | | | | |
|---|---|---|---|---|
| Process step | Line | Volume (column volume) | Linear flow rate (cm/h) | Composition |
| Equilibration | A1 | 5 times | 120 | 100 - 300 mM NaCl, 10 mM EDTA, 20 mM Tris-HCl, pH 7.5 |
| Loading | A2 | 10 - 25 times | Retention time 2 - 4 minutes | Filtered enzyme digestion reaction liquid |
| Postequilibration | A1 | 5 times | 60 | 100 - 300 mM NaCl, 10 mM EDTA, 20 mM Tris-HCl, pH 7.5 |
| Regeneration | B1 | 10 times | 120 | Final concentration 1 M NaOH and 30% isopropanol mixture |

### (8) Concentration and buffer exchange of linear plasmid DNA

The feed liquid collected after the chromatography of linear plasmid DNA was subjected to tangential flow filtration using an ultrafiltration membrane cassette (Pore size 5 KD or 10 KD, Manufacturer: Hangzhou Cobetter Filtration Equipment Co., Ltd., Cat. Nos.: UFELA0010010P, UFELA0005010P, Specifications: 0.01 m², 0.1 m²; a membrane cassette with an appropriate specification was selected according to the actual production scale). The linear plasmid DNA was concentrated to a specified concentration (e.g., 0.5 mg/mL or 1.0 mg/mL), and subjected to buffer exchange into a specified buffer (e.g., sterile water for injection, or Tris-EDTA buffer).

### (9) Sterilization and filtration of linear plasmid DNA

The linear plasmid DNA after the concentration and buffer exchange was subjected to sterilization and filtration using a 0.2 µm filter membrane. Sterilizing filters with a pore size of 0.2 µm were used, with specifications including syringe filters (Manufacturer: Merck Millipore Ltd., Cat. No.: SLGPR33RB), 1000 mL vacuum filtration systems (Manufacturer: Corning Incorporated, Cat. No.: 431098), capsule filters (Manufacturer: Sartorius Stedim Biotech GmbH, Cat. No.: 5445307H7--OO--A). A filter with an appropriate specification was selected according to the volume of the sample after concentration and buffer exchange.

### (10) Linear plasmid DNA filling and storage

After the sterilization and filtration, the finished products of linear plasmid DNA were subjected to filling in an ultra-clean bench or a sterile isolator according to the specified packaging specification, e.g., 1 mL/vial. After the filling, the finished product was stored in an ultra-low temperature refrigerator at -70 ± 10°C.

### (11) Quality detection of linear plasmid DNA

### 1. Detection of intermediate samples and finished products of linear plasmid DNA by agarose gel electrophoresis (AGE)

The intermediate samples and finished products of linear plasmid DNA were detected by using 0.7% agarose gel, with 1×TAE as an electrophoresis buffer. The electrophoresis voltage was 150 V, and the electrophoresis time was 30 minutes. After finishing the electrophoresis, an image was taken using Image Lab software.

### 2. Detection of linear plasmid DNA purity by high-performance liquid chromatography (IEC-HPLC)

Using Agilent 1260 high-performance liquid chromatograph, the purity of the finished products of linear plasmid DNA was detected.

### 3. Detection of linear plasmid DNA by Sanger sequencing verification

Appropriate sequencing primers were designed according to the inserted sequence of interest, and the linear plasmid DNA sample was subjected to Sanger sequencing verification of the whole plasmid.

### 4. Detection of total residual protein in linear plasmid DNA by Nanorange fluorescence

Total residual protein in finished products of linear plasmid DNA was detected by NanoOrange Kit (ThermoFisher, Cat. No.: N10271), and values were read by a microplate reader.

### 5. Detection of residual host DNA (from E. coli) in linear plasmid DNA by fluorescence quantitative PCR method (qPCR)

The residual host DNA (from *E. coli)* in linear plasmid DNA were detected using a real-time fluorescence quantitative PCR instrument (ThermoFisher, model: 7500) and residual *E. coli* DNA detection kit (Huzhou Shenke Biotechnology Co., Ltd., Cat. No.: SK030202E100).

### 6. Detection of bacterial endotoxin in linear plasmid DNA by gel-clot test method (LAL)

Bacterial endotoxin in linear plasmid DNA was detected by gel-clot test method using Limulus Amebocyte Lysate.

### 7. Direct inoculation method to detect whether linear plasmid DNA was sterile

The linear plasmid DNA sample was pipetted by a disposable sterile syringe or sterilized pipette, inoculated into 100 mL of a thioglycolate FTM culture medium, and cultured at 30-35°C for 14 days, and it was detected whether the linear plasmid DNA sample was sterile.

According to the method in Example 1, two batches of linear plasmid DNA were prepared. The experimental results for the first batch of linear plasmid DNA are shown in Tables 1-2 and FIGs. 2-4. The experimental results for the second batch of linear plasmid DNA are shown in Tables 3-4 and FIGs. 5-7.

It can be seen from the results:
1. This linear plasmid DNA production process is very stable. The scale of circular plasmids for digestion is gradually enlarged from 30 mg to 100 mg, and no abnormal deviation occurs in the production process. This process can also satisfy the need of linear plasmid DNA production on the digestion scale of more than 100 mg of the circular plasmids.
2. The total yield% of this linear plasmid DNA production process is high. The total yield% for the linear plasmid DNA produced in two batches was 45%-47%.
3. This linear plasmid DNA production process has a short production process cycle. For the same production scale, it is estimated that 3-4 days can be saved per production batch.
4. The finished products of linear plasmid DNA produced by this process have purity and quality both meeting the requirements of the pharmacopoeia, and can be used for clinical research and commercial use in gene therapy and other fields.

**Table 1 Production data of the first batch of linear plasmid DNA**

| Batch No. | Natrix Q loading amount in mg | Natrix Q chromatograp hy yield% | Digestion scale in mg | Core 700 chromatogra phy yield% | Linear plasmid DNA yield in mg | Total yield % |
|---|---|---|---|---|---|---|
| First batch | 120.00 | 65.38 | 30.00 | 86.05 | 22.81 | 47.44 |

**Table 2 Detection data of the first batch of linear plasmid DNA**

| Quality standard of linear plasmid DNA | | | | Detection results |
|---|---|---|---|---|
| No. | Key quality attributes | Methods | Standard requirements | First batch |
| 1 | Appearance | Visual observation method | Colourless clear liquid | Colourless clear liquid |
| 2 | DNA purity | Ultraviolet spectrophotometry (260 nm/280 nm) | 1.8-2.0 | 1.85 |
| 3 | Concentrations | Ultraviolet spectrophotometry (260 nm) | 1.0 ± 0.1 mg/mL | 1.00 |
| 4 | pH | pH meter | 7.0-8.5 | 7.5 |
| 5 | DNA homogeneity | IEC-HPLC | ≥ 90% | 97.00 |
| 6 | Whole plasmid sequencing | Sanger | Consistent with the theoretical sequence | Consistent with the theoretical sequence |
| 7 | Total residual protein | Nanorange | < 10 µg/mL | <1 µg/mL |
| 8 | Residual *Escherichia coli* host DNA | qPCR | ≤ 1.0% | 0.23 |
| 9 | Bacterial endotoxin | TAL | < 10 EU/mg | 9.150 EU/mg |
| 10 | Microbial limit | Membrane filtration method | < 10 CFU/mL | < 1 CFU/mL |

**Table 3 Summary of production data of the second batch of linear plasmid DNA**

| Batch No. | Natrix Q loading amount in mg | Natrix Q chromatogr aphy yield% | Digestion scale in mg | Core 700 chromatogr aphy yield% | Linear plasmid DNA yield in mg | Total yield % |
|---|---|---|---|---|---|---|
| Second batch | 120.00 | 96.28 | 100.00 | 83.42 | 46.73 | 44.99 |

**Table 4 Summary of detection data of the second batch of linear plasmid DNA**

| Quality standard of linear plasmid DNA | | | | Detection results |
|---|---|---|---|---|
| No. | Key quality attributes | Methods | Standard requirements | Second batch |
| 1 | Appearance | Visual observation method | Colourless clear liquid | Colourless clear liquid |
| 2 | DNA purity | Ultraviolet spectrophotometry (260 nm/280 nm) | 1.8-2.0 | 1.89 |
| 3 | Concentrations | Ultraviolet spectrophotometry (260 nm) | 1.0 ± 0.1 mg/mL | 1.00 |
| 4 | pH | pH meter | 7.0-8.5 | 7.6 |
| 5 | DNA homogeneity | IEC-HPLC | ≥ 90% | 96.00 |
| 6 | Whole plasmid sequencing | Sanger | Consistent with the theoretical sequence | Consistent with the theoretical sequence |
| 7 | Total residual protein | Nanorange | < 10 µg/mL | < 2.88 µg/mL |
| 8 | Residual *Escherichia coli* host DNA | qPCR | ≤ 1.0% | 0.70 |
| 9 | Bacterial endotoxin | TAL | < 10 EU/mg | 4.707 EU/mg |
| 10 | Microbial limit | Membrane filtration method | < 10 CFU/mL | < 1 CFU/mL |

### Example 2 Method for preparing and purifying LcDNA

Reference can be made to steps (1)-(5) of Example 1 to obtain circular plasmids.

### (6) Three-step digestion of circular plasmids

The Natrix Q eluate was first adjusted to pH 7.0 - 8.0 using a 3M Tris buffer, and then subjected to three-step digestion sequentially:
Step I: Prokaryotic telomerase TelN digestion (using TelN, digestion system 1000 mL as an example). After sequentially adding and mixing thoroughly the components in the table below, the mixture was incubated in a constant temperature water bath at 30°C for 3 hours, followed by treatment of digestion at 75°C for 15 - 30 minutes.

| Component | Addition amount |
|---|---|
| Digestion buffer | 100 mL (buffer components include 200 mM Tris-HCl, 100 mM (NH4)2SO4, 100 mM KCl, 20 mM MgSO4, and 1% Triton^{®} X-100) |
| Natrix Q eluate | 40 mg (e.g., concentration 0.2 mg/mL, i.e., 200 mL) |
| TelN | 6.4 - 8.0 mL (unit enzyme activity 50000 U/mL, digestion efficiency 8000-10000 U/mg plasmid) |
| Water for injection | Add water for injection to adjust the salt concentration in the digestion system to less than 0.1 M |

Step II: Restriction endonuclease digestion (using KpnI-HF and SalI-HF double digestion, digestion system 1200 mL as an example). After sequentially adding and mixing thoroughly the components in the table below, the mixture was incubated in a constant temperature water bath at 37°C for 2 hours, followed by treatment of digestion at 75°C for 15 - 30 minutes.

| Component | Addition amount |
|---|---|
| Digestion buffer | 120 mL |
| Enzyme digestion reaction liquid from Step I | 1000 mL |
| KpnI-HF | 2 - 4 mL (unit enzyme activity 20000 U/mL, digestion efficiency 1000-2000 U/mg plasmid) |
| Sall-HF | 2 - 4 mL (unit enzyme activity 20000 U/mL, digestion efficiency 1000-2000 U/mg plasmid) |
| Water for injection | Add up to 1200 mL |

Step III: Exonuclease digestion (using Exo III, digestion system 1400 mL as an example). After sequentially adding and mixing thoroughly the components in the table below, the mixture was incubated in a constant temperature water bath at 37°C for 4 - 12 hours. To terminate the digestion, EDTA at a final concentration of 10 mM was first added, followed by reaction at 75°C for 10 - 30 minutes.

| Component | Addition amount |
|---|---|
| Digestion buffer | 140 mL |
| Enzyme digestion reaction liquid from Step II | 1200 mL |
| Exo III | 1.2 - 2.4 mL (unit enzyme activity 100000 U/mL, digestion efficiency 3000-6000 U/mg plasmid) |
| Water for injection | Add up to 1400 mL |

### (7) Chromatographic purification of LcDNA

The solution obtained in step (6) was first filtered through a 0.2 µm filter membrane, and the filtrate was collected and then loaded onto a chromatography column containing the composite resin filler Capto Core 700 (Manufacturer: Cytiva, Cat. No.: 17-5481-03). The value at UV260 or UV280 was monitored, and fractions with values greater than or equal to 30 mAU were collected. The parameters of the chromatography process are shown in the table below:

| Capto Core 700 chromatography | | | | |
|---|---|---|---|---|
| Process step | Line | Volume (column volume) | Linear flow rate (cm/h) | Composition |
| Equilibration | A1 | 5 times | 120 | 100 - 300 mM NaCl, 10 mM EDTA, 20 mM Tris-HCl, pH 7.5 |
| Loading | A2 | 10 - 25 times | Retention time 2 - 4 minutes | Filtered enzyme digestion reaction liquid |
| Postequilibration | A1 | 5 times | 60 | Same as A1 above |
| Regeneration | B1 | 10 times | 120 | Final concentration 1 M NaOH and 30% isopropanol mixture |

### (8) Concentration and buffer exchange of LcDNA

The feed liquid collected after the chromatography of LcDNA was subjected to tangential flow filtration using an ultrafiltration membrane cassette (Pore size 5 KD or 10 KD, Manufacturer: Hangzhou Cobetter Filtration Equipment Co., Ltd., Cat. Nos.: UFELA0010010P, UFELA0005010P, Specifications: 0.01 m², 0.1 m²; a membrane cassette with an appropriate specification was selected according to the actual production scale). The LcDNA was concentrated to a specified concentration (e.g., 0.5 mg/mL or 1.0 mg/mL), and subjected to buffer exchange into a specified buffer (e.g., sterile water for injection, or Tris EDTA buffer).

### (9) Sterilization and filtration of LcDNA

The LcDNA after the concentration and buffer exchange was subjected to sterilization and filtration using a 0.2 µm filter membrane. Sterilizing filters with a pore size of 0.2 µm were used, with specifications including syringe filters (Manufacturer: Merck Millipore Ltd., Cat. No.: SLGPR33RB), 1000 mL vacuum filtration systems (Manufacturer: Corning Incorporated, Cat. No.: 431098), capsule filters (Manufacturer: Sartorius Stedim Biotech GmbH, Cat. No.: 5445307H7--OO--A). A filter with an appropriate specification was selected according to the volume of the sample after concentration and buffer exchange.

### (10) LcDNA filling and storage

After the sterilization and filtration, the finished products of LcDNA were subjected to filling in an ultra-clean bench or a sterile isolator according to the specified packaging specification, e.g., 1 mL/vial. After the filling, the finished product was stored in an ultra-low temperature refrigerator at -70 ± 10°C.

### (11) Quality detection of LcDNA

### 1. Detection of intermediate samples and finished products of LcDNA by agarose gel electrophoresis (AGE)

The intermediate samples and finished products of LcDNA were detected by using 0.7% agarose gel, with 1×TAE as an electrophoresis buffer. The electrophoresis voltage was 150 V, and the electrophoresis time was 30 minutes. After finishing the electrophoresis, an image was taken using Image Lab software.

### 2. Detection of LcDNA purity by high-performance liquid chromatography (IEC-HPLC)

Using Agilent 1260 high-performance liquid chromatograph, the purity of the finished products of LcDNA was detected.

### 3. Detection of LcDNA by Sanger sequencing verification

Appropriate sequencing primers were designed according to the inserted sequence of interest, and the LcDNA sample was subjected to Sanger sequencing verification of the whole plasmid.

### 4. Detection of total residual protein in LcDNA by Nanorange fluorescence

Total residual protein in finished products of LcDNA was detected by NanoOrange Kit (ThermoFisher, Cat. No.: N10271), and values were read by a microplate reader.

### 5. Detection of residual host DNA (from E. coli) in LcDNA by fluorescence quantitative PCR method (qPCR)

The residual host DNA (from *E. coli)* in LcDNA were detected using a real-time fluorescence quantitative PCR instrument (ThermoFisher, model: 7500) and residual *E. coli* DNA detection kit (Huzhou Shenke Biotechnology Co., Ltd., Cat. No.: SK030202E100).

### 6. Detection of bacterial endotoxin in LcDNA by gel-clot test method (LAL)

Bacterial endotoxin in LcDNA was detected by gel-clot test method using Limulus Amebocyte Lysate.

### 7. Direct inoculation method to detect whether LcDNA was sterile

The LcDNA sample was pipetted by a disposable sterile syringe or sterilized pipette, inoculated into 100 mL of a thioglycolate FTM culture medium, and cultured at 30-35°C for 14 days, and it was detected whether the LcDNA sample was sterile.

According to the method in Example 2, three batches of LcDNA were prepared. The experimental results for the first batch of LcDNA are shown in Tables 5-6 and FIGs. 8-10. The experimental results for the second batch of LcDNA are shown in Tables 7-8 and FIGs. 11-13. The experimental results for the third batch of LcDNA are shown in Tables 9-10 and FIGs. 14-16.

It can be seen from the results:
1. This LcDNA production process is very stable, with no abnormal deviations occurring during the process of production.
2. The total yield% of this LcDNA production process is high. The total yield% for the LcDNA produced in 3 batches was 22%-24%.
3. This LcDNA production process has a short production process cycle. For the same production scale, it is estimated that 3-4 days can be saved per production batch.
4. The finished products of LcDNA produced by this process have purity and quality both meeting the requirements of the pharmacopoeia, and can be used for clinical research and commercial use in gene therapy and other fields.

**Table 5 Production data of the first batch of LcDNA**

| Batch No. | Natrix Q loading amount in mg | Natrix Q chromatogra phy yield% | Digestion scale in mg | Core 700 chromatograp hy yield% | LcDNA yield in mg | Total yield % |
|---|---|---|---|---|---|---|
| First batch | 120.00 | 71.90 | 50.00 | 95.71 | 17.00 | 24.45 |

**Table 6 Detection data of the first batch of LcDNA**

| LcDNA quality standard | | | | Detection results |
|---|---|---|---|---|
| No. | Key quality attributes | Methods | Standard requirements | First batch |
| 1 | Appearance | Visual observation method | Colourless clear liquid | Colourless clear liquid |
| 2 | DNA purity | Ultraviolet spectrophotometry (260 nm/280 nm) | 1.8-2.0 | 1.90 |
| 3 | Concentrations | Ultraviolet spectrophotometry (260 nm) | 1.0 ± 0.1 mg/mL | 1.00 |
| 4 | pH | pH meter | 7.0-8.5 | 7.70 |
| 5 | DNA homogeneity | IEC-HPLC | ≥ 90% | 100.00 |
| 6 | Whole plasmid sequencing | Sanger | Consistent with the theoretical sequence | Consistent with the theoretical sequence |
| 7 | Total residual protein | Nanorange | < 10 µg/mL | 3.6 µg/mg |
| 8 | Residual *Escherichia coli* host DNA | qPCR | ≤ 1.0% | 0.07% |
| 9 | Bacterial endotoxin | TAL | < 10 EU/mg | < 1.000 EU/mg |
| 10 | Sterility detection | Culture method | Absence of microbial growth | Absence of microbial growth |

**Table 7 Production data of the second batch of LcDNA**

| Batch No. | Natrix Q loading amount in mg | Natrix Q chromatograp hy yield% | Digestio n scale in mg | Core 700 chromatograp hy yield% | LcDNA yield in mg | Total yield % |
|---|---|---|---|---|---|---|
| Second batch | 120.00 | 63.55 | 50.00 | 113.17 | 19.00 | 24.15 |

**Table 8 Detection data of the second batch of LcDNA**

| LcDNA quality standard | | | | Detection results |
|---|---|---|---|---|
| No. | Key quality attributes | Methods | Standard requirements | Second batch |
| 1 | Appearance | Visual observation method | Colourless clear liquid | Colourless clear liquid |
| 2 | DNA purity | Ultraviolet spectrophotometry (260 nm/280 nm) | 1.8-2.0 | 1.90 |
| 3 | Concentrations | Ultraviolet spectrophotometry (260 nm) | 1.0 ± 0.1 mg/mL | 1.00 |
| 4 | pH | pH meter | 7.0-8.5 | 7.7 |
| 5 | DNA homogeneity | IEC-HPLC | ≥ 90% | 100.00 |
| 6 | Whole plasmid sequencing | Sanger | Consistent with the theoretical sequence | Consistent with the theoretical sequence |
| 7 | Total residual protein | Nanorange | < 10 µg/mL | 4.0 µg/mg |
| 8 | Residual *Escherichia coli* host DNA | qPCR | ≤ 1.0% | 0.02% |
| 9 | Bacterial endotoxin | TAL | < 10 EU/mg | < 1.000 EU/mg |
| 10 | Sterility detection | Culture method | Absence of microbial growth | Absence of microbial growth |

**Table 9 Production data of the third batch of LcDNA**

| Batch No. | Natrix Q loading amount in mg | Natrix Q chromatograp hy yield% | Digestion scale in mg | Core 700 chromatogra phy yield% | LcDNA yield in mg | Total yield% |
|---|---|---|---|---|---|---|
| Third batch | 120.00 | 66.60 | 50.00 | 102.42 | 17.00 | 22.64 |

**Table 10 Detection data of the third batch of LcDNA**

| LcDNA quality standard | | | | Detection results |
|---|---|---|---|---|
| No. | Key quality attributes | Methods | Standard requirements | Third batch |
| 1 | Appearance | Visual observation method | Colourless clear liquid | Colourless clear liquid |
| 2 | DNA purity | Ultraviolet spectrophotometry (260 nm/280 nm) | 1.8-2.0 | 1.90 |
| 3 | Concentrations | Ultraviolet spectrophotometry (260 nm) | 1.0 ± 0.1 mg/mL | 1.00 |
| 4 | pH | pH meter | 7.0-8.5 | 7.8 |
| 5 | DNA homogeneity | IEC-HPLC | ≥ 90% | 99.00 |
| 6 | Whole plasmid sequencing | Sanger | Consistent with the theoretical sequence | Consistent with the theoretical sequence |
| 7 | Total residual protein | Nanorange | < 10 µg/mL | <3.2 µg/mg |
| 8 | Residual *Escherichia coli* host DNA | qPCR | ≤ 1.0% | 0.12% |
| 9 | Bacterial endotoxin | TAL | < 10 EU/mg | < 10 EU/mg |
| 10 | Sterility detection | Culture method | Absence of microbial growth | Absence of microbial growth |

The embodiments of the present invention are not limited to the above examples, and various modifications and improvements in forms and details may be made to the present invention by those of ordinary skill in the art without departing from the spirit and scope of the present invention, and these modifications and improvements are all considered to fall within the scope of protection of the present invention.

## Claims

1. A method for purifying a nucleic acid molecule, **characterized in that** the method comprises:
1) providing a suspension comprising the nucleic acid molecule; and
2) loading the suspension from step 1 onto a porous polyacrylamide membrane and performing capture, to obtain the nucleic acid molecule.

2. The method according to claim 1, **characterized in that** the suspension in step 1) comprises a NaCl solution at a final concentration of 50 - 150 mM.

3. The method according to claim 1 or 2, **characterized in that** the method further comprises, prior to the loading of step 2), a step of adding an equilibration buffer to the porous polyacrylamide membrane.

4. The method according to any one of claims 1-3, **characterized in that** the method further comprises, after the loading of step 2), a step of adding the equilibration buffer to the porous polyacrylamide membrane.

5. The method according to claim 3 or 4, **characterized in that** the equilibration buffer is a mixed solution of 1M KAc and 150 mM NaCl.

6. The method according to any one of claims 1-5, **characterized in that** the method further comprises, after the capture of step 2), a step of performing gradient elution on the obtained nucleic acid molecule and collecting an eluate comprising the nucleic acid molecule.

7. The method according to claim 6, **characterized in that** a buffer for the gradient elution is a mixed solution of 100 mM Tris and 1M NaCl in an elution gradient of 20%.

8. The method according to claim 6, **characterized in that** the collecting an eluate is collecting an eluate when the UV value at UV280 or UV260 is greater than or equal to 200 mAU.

9. The method according to any one of claims 1-8, **characterized in that** the porous polyacrylamide membrane is a high-density strong anion porous polyacrylamide membrane, preferably a Natrix Q membrane.

10. The method according to any one of claims 1-9, **characterized in that** the nucleic acid molecule is a circular nucleic acid molecule, and preferably circular DNA.

11. The method according to claim 10, **characterized in that** the circular nucleic acid molecule is a plasmid comprising at least one restriction endonuclease digestion site.

12. The method according to claim 11, **characterized in that** the circular nucleic acid molecule is a plasmid, the plasmid comprises two prokaryotic telomerase digestion sites which are connected in tandem in the same direction, both ends of the prokaryotic telomerase digestion site are independently provided with one or more restriction endonuclease digestion sites, and a gene of interest is inserted between the prokaryotic telomerase digestion sites.

13. The method according to claim 11, **characterized in that** the restriction endonuclease digestion site at each end of the plasmid is selected from any one or more of digestion sites of: AccI, AflII, Agel, ApaI, AscI, AvrII, BamHI, BglI, BglII, BsaI, BspQI, BstBI, BsteII, ClaI, EcoNI, EcoRI, EcoRV, FseI, HindIII, KpnI, MfeI, MluI, NcoI, NdeI, NheI, NotI, PacI, PstI, Pvul, PvuII, SacI, SacII, Sall, ScaI, SmaI, SpeI, StuI, SwaI, XbaI, XhoI and XmaI.

14. The method according to claim 13, **characterized in that** the restriction endonuclease digestion site is selected from any one or more of digestion sites of: HindIII, Sall, SmaI, BamHI, EcoRV, XbaI, KpnI and EcoRI.

15. A method for preparing LcDNA, **characterized in that** the method comprises:
a) providing a plasmid obtained by the method according to claim 12, and preforming digestion with a prokaryotic telomerase;
b) preforming digestion with at least two restriction endonucleases, and preforming further enzymatic digestion with an exonuclease; and
c) polishing the LcDNA containing a gene of interest.

16. A method for preparing linear DNA, **characterized in that** the method comprises:
a) providing a plasmid obtained by the method according to claim 11, and preforming digestion with a restriction endonuclease; and
b) polishing the linear DNA containing a gene of interest.

17. The method according to claim 15 or 16, **characterized in that** the method further comprises, prior to the step of digestion, adjusting the pH of the eluate to pH 7.0-8.0 using a 3M Tris buffer.

18. The method according to any one of claims 15-17, **characterized in that** the polishing comprises using a composite resin filler Capto Core 700 for chromatography.

19. The method according to claim 18, **characterized in that** the chromatography comprises:
a) adding an equilibration buffer to the resin; and
b) loading an enzyme digestion reaction liquid for chromatography and collecting a flow-through liquid;
and the equilibration buffer is a mixed solution of 100 - 300 mM NaCl, 10 mM EDTA and 20 mM Tris-HCl.

20. The method according to any one of claims 15-19, **characterized in that** the method further comprises, after the polishing, at least one process selected from concentration, sterilization and filtration, and filling.

21. The LcDNA prepared according to claim 15 or the linear DNA prepared according to claim 16 for use in the preparation of mRNA.
